# EUROPEAN PATENT SPECIFICATION

(11) **EP 0 550 067 B1**
(45) Date of publication and mention of the grant of the patent: **18.09.1996**
(21) Application number: 92122122.2
(22) Date of filing: 30.12.1992
(51) Int. Cl.: A23L 1/22, A61K 7/46, C11B 9/00

(54) **High load spray dry encapsulation**
Verkapselung einer hohen Ladung durch Sprühtrocknung
Encapsulation d'une forte charge par séchage par pulvérisation

(30) Priority: 30.12.1991 US 813172
(43) Date of publication of application: 07.07.1993
(73) Proprietor: HERCULES INCORPORATED, Wilmington Delaware 19894-0001 (US)
(72) Inventor: Hussain, Zahera Jabeen, Hyde Park, New York 12538 (US)
(74) Representative: Keller, Günter, Dr.

(56) References cited:
- WO-A-85/03414
- FR-A- 2 233 095
- US-A- 2 661 349
- US-A- 3 091 567
- US-A- 3 455 838

## Description

This invention relates to a method for encapsulating oils such as fragrance or flavoring oils in a water-sensitive cellular solid matrix, especially in particulate form.

U.S. Patent 3,971,852 of Brenner discloses a method for encapsulating oils in particles of a solid water-sensitive, preferably water-soluble, protective matrix that isolates the oils until they are released for use by exposure of the particles to moisture.

The matrix-forming encapsulation materials include mixtures of polysaccharides and polyhydroxy compounds that can form aqueous emulsions with the oil. They have a plastic or flowable state over a substantial temperature range over which water is readily removed from the emulsion and they become, on removal of moisture, a cellular matrix of the polysaccharide and polyhydroxy materials in solid form with cells containing the oil.

The known process for making these compositions, as disclosed in the patent, comprises forming an aqueous-phase containing a polysaccharide and a polyhydroxy compound (amounting to a solids content of about 25%), and adjuncts such as emulsifiers and the like, emulsifying the oil in the aqueous phase, and finally removing the moisture by spray drying, slab drying, belt drying, or dehydration by a dehydrating agent, preferably at a temperature that keeps the matrix plastic until it is dry.

The polysaccharides employed in admixture with polyhydroxy compounds in the known processes are solids characterized by solubility in water and by at least partial solubility in, or capability of at least partially dissolving, the polyhydroxy compound within the ranges of proportions used. Examples of the polysaccharides are natural gums such as gum arabic, dextranized or hydrolyzed starches, and other starch derivatives. The polyhydroxy compounds may be polyhydroxy alcohols, plant type sugars such as maltose and sucrose, lactones, monoethers, and acetals. The modified products of starch usually form colloidal solutions.

The said patent claims efficient encapsulation up to 80% by volume and "surface oil" not substantially above 5%. (Encapsulation efficiency is in inverse relationship to the quantity of "surface oil" extractable in Freon in a period of 10 minutes and "extended surface oil" extractable in Freon in a period of 72 hours. However, with a relatively high oil content ("loading"), the known process fails to provide efficient encapsulation oil recovery, with excessive oil loss during drying, and extractable oils as high as 10-24% when the encapsulated oil content exceeds 60% by weight.

Experience with the known method in the sixteen years since the said patent issued has demonstrated that a need exists for an improved method that actually and reliably realizes 70-80% of the encapsulate (oil phase) in an occlusive matrix, with extractable oil below 3% at an oil content of 70% by weight. The need is also for stable emulsions that can be processed to form free-flowing powders upon drying, while allowing for unavoidable variations in the encapsulation procedures, and accommodating a wide range of encapsulates (only orange oil is disclosed as the encapsulate in the said U.S. Patent 3,971,852).

The present invention is a method for encapsulating oils such as fragrance or flavoring oils in a water-sensitive cellular solid matrix, comprising drying an aqueous emulsion containing the oil to be encapsulated, a non-cross-linked lipophilically modified starch that undergoes cross-linking under the drying conditions, and a polyhydroxy compound that forms with the polysaccharide material a continuous aqueous phase in which oil is dispersible as a discontinuous phase.

It is well known that films of cross-linked starches have better structural strength and barrier properties than their corresponding uncrosslinked counterparts. However, starch that is already conventionally crosslinked (e.g., using a common crosslinking agent such as formaldehyde) cannot be used in the process according to the invention.

The integrity and strength of the matrix material may be changed by adjusting the composition of the matrix material, thus providing convenient control over the properties of the matrix. For instance, both increasing the ratio of solids to water in the emulsion and increasing the pH of the emulsion, increase the integrity and strength of the matrix material. The use of less water not only reduces drying time, but also unexpectably results in emulsions having better stability. The proportions are chosen to give the desired oil loading in an emulsion having from 40 to 80% solids.

Also, according to a preferred aspect of the invention, the integrity and strength of the matrix material is increased by incorporating pliability-increasing polymeric plasticizers (often called film formers) as emulsifiers for the oil in the aqueous phase, most preferably silicone-based emulsifiers such as those available from Dow Corning, particularly DC 929 Emulsion®, which is an amino-functional cationic emulsion of polydimethylsiloxane. The pliability-increasing effect of these materials minimizes the rupture of dried particles when subjected to mechanical shear.

Although the preferred procedure for drying the emulsion mixture is by conventional spray drying to form a particulate product, it is possible to prepare the product for use in other forms, such as by coating a container internally with a layer of the emulsion and then drying it. The encapsulate can be released by filling the container with water. Similarly, carbon-less carbon paper may be prepared by coating directly with the emulsion and drying. If the products of the invention are prepared by drying procedures other than spraying, e.g., by drying on belts, drums, and similar surfaces, the product may be ground to the desired particle size.

The powder produced by spray drying in accordance with the invention can be incorporated into conventional compositions such as cosmetics, toiletries, household products such as cleansers, personal care products such as disposable baby diapers, feminine napkins, and paper towels.

The invention involves cross-linking a lipophilically modified starch, such as a succinate or substituted succinate starch or a linoleate or substituted linoleate starch, by drying an aqueous emulsion containing the oil to be encapsulated, the modified starch and a polyhydroxy compound that forms with the starch a continuous aqueous phase in which oil is dispersible as a discontinuous phase. The polysaccharide may possess inherent emulsifying properties or may be supplemented by a known emulsifying agent.

More specifically, the said method comprises preparing an aqueous solution of the non-cross-linked modified starch and the polyhydroxy compound, adding the oil to the resulting solution to form an an oil-in-water emulsion, and drying the emulsion.

Preferably, from 10 % to 90% of the modified starch and from 90% to 10% of the polyhydroxy compound, based on the total solids weight, are dissolved with agitation at high speed in an amount of water such that the emulsion contains at least 40% solids. Also preferably, the pH of the emulsion, which is inherently about 2.9 to 3.3, is adjusted to 4.5 to 5.5 before the drying stage when crosslinking occurs. Preferably, more than 50% of the crosslinkable bonds of the polysaccharide are crosslinked.

The oil, preferably containing a conventional antioxidant, such as 0.01 to 0.05% butylated hydroxy toluene or anisole to retard rancidity, is added to the emulsion while continuing high speed agitation, preferably until from 10 parts to 70 parts by weight are incorporated in the emulsion and the average droplet size of the oil is from 0.5 to 2 microns. The most preferred antioxidant is 0.01% butylated hydroxy toluene.

The proportions of oil to encapsulate in the dried matrix may vary widely as desired, and typically, the fragrance oil will amount to from about 5 to about 80% by volume of the encapsulated fragrance matrix. Preferably the amount of the oil encapsulated in the matrix is at least 65% by weight of the oil and matrix combination.

The modified starch is an ungelatinized starch acid ester of a substituted dicarboxylic acid having the formula: 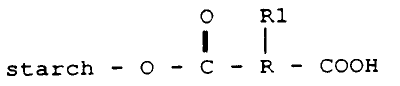 in which R is a radical selected from the class consisting of dimethylene and trimethylene and R1 is a hydrocarbon substituent selected from the class consisting of alkyl, alkenyl, aralkyl groups, the ungelatinized starch-acid ester being the reaction product of an ungelatinized starch and a substituted cyclic dicarboxylic acid anhydride having the formula: 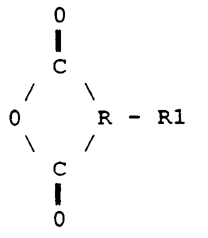 in which R and R1 represent the defined substituent groups. Examples of such anhydrides are the substituted succinic and glutaric acid anhydrides. Other useful polysaccharides include products derived from dextrinized starch, and hydrolyzed starch.

Preferably, the modified starch is selected from the group consisting of succinate starch, substituted succinate starch, linoleate starch, and substituted linoleate starch.

If the polysaccharide possesses insufficient emulsifying properties inherently, it is conventionally supplemented by a known emulsifying agent, such as sodium diisooctyl sulfosuccinate or sodium caseinate in the range of 0.1 to 10%, based on the weight of polysaccharide in the mixture.

The preferred polyhydroxy compounds are: alcohols, sugars from plant sources and those containing other functional groups. Polyhydroxy alcohols include glycerine, sorbitol, mannitol, erythritol and ribitol. The sugars from plant sources, include monosaccharides such as glucose, disaccharides such as maltose and sucrose, trisaccharides such as raffinose, and ketosaccharides such as fructose. These will be referred to as plant-type sugars whether actually derived from plants or produced synthetically. Polyhydroxy compounds containing other functional groups include glucuronolactone (lactone), sorbitan and mannitan (monoethers) and methyl-glucopyranoside (acetal). Preferably the polyhydroxy compounds form with the polysaccharide material a liquid melt that softens within the range of from -1 to 38°C (30 to 100°F).

The emulsion is dried in a conventional spray dryer, such as a Standard Anhydro Laboratory spray drier Size # 1. The inlet and outlet temperatures may be the conventional ones for the matrix material used, for instance, 180° and 80°C respectively, but are preferably 200 and 100°C respectively, but are preferably 200 and 100°C respectively. Preferably, the particles produced have a diameter no greater than 75 · so that they readily pass through a 200 mesh screen. The plasticity of the surface of the particles formed from the emulsion diminishes as water is removed through a drying operation, and the particles formed are in a solid state at the normal ambient temperatures of use.

Certain starch degradation products such as dextrinized starch, which are suitable polysaccharides for use in the invention, contain a wide spectrum of saccharides of different molecular weights, including a sufficient proportion in the polysaccharide molecular weight range to be good encapsulants and varying proportions of lower saccharides such as mono-, di- and trisaccharides that are polyhydroxy compounds. Since the proportion of lower saccharides in the starch degradation products is usually too low to satisfy the requirements for the polyhydroxy compounds to form an effective eutectic composition, as well known to those skilled in the art, while being large enough to affect significantly the amount of added polyhydroxy compounds required to obtain the proper balance of polysaccharide and polyhydroxy compounds, it may be necessary to make conventional adjustments in the proportion of added polyhydroxy from 40 to 60% solids, preferably about 60%.

Also, the invention contemplates the addition of a silicone-based emulsifier, such as the above-mentioned DC 929® cationic emulsifier. It is preferably used in an amount of from 1 to 5% to serve as a plasticizer that increases the integrity of the matrix. Examples of other polymeric plasticizers that can be used as emulsifiers are known film formers such as hydroxypropylcellulose emulsifiers, for example those sold by Aqualon Company under the trademark Klucel®, or GAFQUAT® polyvinylpyrrolidones from GAF.

Preferably, the polysaccharide or polysaccharide-emulsifier combination is such that when dissolved in water with the polyhydroxy compound, the aqueous phase is capable of emulsifying the oil to form the dispersed phase of an oil-in-water emulsion with the oil globules having diameters largely within the preferred range of about 0.5 to 2.0 µm and has sufficient stability not to invert or coalesce prior to moisture removal.

The oils that can be encapsulated in accordance with the present invention include nonvolatile as well as volatile oils. They are insoluble but dispersible (emulsifiable) in water and they may be volatile or nonvolatile under drying conditions that include elevated temperatures and low relative humidity. They are usually liquid at the temperature of the emulsion but any non-liquid oil that can be readily broken up into tiny particles in an emulsifying machine producing high shear, such as petroleum jelly, can be successfully encapsulated for use in the process of the invention.

The viscosity as determined by the Brookfield Model LVT Viscometer is from 50 to 150 centipoise at 35 C. The proportions are chosen to give the selected oil loading in an emulsion having from 40 t0 60% solids. The mixture is spray dried in a conventional spray dryer, such as a Standard Anhydro Laboratory spray drier Size # 1. The inlet and outlet temperatures may be conventional for the matrix material used, for instance, 80° and 180°C respectively.

Preferably, the particles produced have a diameter no greater than 75µm that readily pass through a 200 mesh screen. The finished powder can be analyzed by wide band NMR to determine the percentages of total, TO, and extractable surface oil, SO, and the moisture content can be determined by the conventional Karl Fischer Method of Analysis for water. The surface oil measured after a friability test, FSO, is an indication of the strength of the matrix. The friability test is carried out by vigorously mixing 15g of the encapsulation with 0.5 g of Syloid 244,191300 for one hour and then measuring the oil released with the standard NMR test. The level of "extended surface oil", Ex. SO, which is extractable over 72 hours is a measure of the efficiency of encapsulation, and if not over the preferred level of 2%, it indicates a superior degree of encapsulation.

Following are comparative examples that clarify the advantages of the invention and examples that illustrate the invention. In all cases temperatures are given in degrees centigrade and proportions are by weight unless specified to be by volume.

### Comparative Example 1

This Example is a control experiment to demonstrate the results obtained by known processes, as exemplified by U.S. Patent 3,971,852 of Brenner, with an oil loading of 50% in an emulsion with 50% solids at its inherent pH of about 3, and spray dried at inlet/outlet temperatures of 180/80°C.

A solution of encapsulant comprising of 48 parts of modified starch, (obtained from National Starch Company under the trademark Capsul®) and 12 parts of polyhydroxy alcohol is prepared by dissolving them with agitation at high speed in a household type Waring blender in 100 parts of water (a total of 150 parts). A fragrance oil available from Hercules Incorporated under the trade name Powder floral fragrance (containing 0.01% butylated hydroxy toluene to retard rancidity) is slowly added to the resulting solution until 90 parts by weight are incorporated while continuing high speed agitation for 3 minutes, until an oil/water emulsion has been formed with an average droplet size of 0.5 µms. The viscosity as determined by the Brookfield Model LVT Viscometer is 70 mPas (70 centipoise) at 35°C. The proportions are chosen to give an oil loading of 50% by weight in an emulsion having 50% solids. The mixture is spray dried in a Standard Anhydro Laboratory spray drier Size # 1 maintained at an inlet temperature of 180°C and outlet temperature of 80°C. There is collected 130 parts of the powder readily passing through 200 mesh screen, representing a particle diameter of no greater than 75µm. Analysis of the finished powder by wide band NMR indicated the % total and surface oil (extractable in Freon in a period of 10 minutes) to be 57.6 and 0.1 respectively. The moisture content was 1.1%. Extended surface oil extractable in Freon-11 in a period of 72 hours was 1.2%, indicating encapsulation efficiency lower than that alleged in Example 1 of the said U.S. Patent of Brenner, which indicated extractable oil at 0.2%.

### Comparative Example 2

The procedure of Example 1 was repeated with total oil loading increased to 60%. The results are shown in the Table below, along with the results of Comparative Example 1.

### Comparative Example 3

An emulsion prepared by the procedure of Comparative Example 1, but with the total oil loading raised to 70%, gave clearly inferior encapsulation, as indicated by the results shown in the Table, including surface oil at 5.2%, friability surface oil at 18.6%, and extended surface oil at 15.7%.

The following examples 4 and 5 indicate the effects of increasing the solids level to 60%, with higher inlet and outlet temperatures, without crosslinking.

### Example 4

The emulsion from Example 2 (60% total oil loading but in a 60% solids emulsion) was spray dried at higher inlet and outlet temperatures of 200°C and 100°C respectively. The efficiency of encapsulation was improved over that in Example 2, as indicated by a surface oil level of 0.4%, with a total oil level at 59.2% by weight. The results are summarized in the Table. The product dissolved more readily in cold water than the powder obtained in Example 1.

### Example 5

An emulsion with the same matrix material as in Example 3, but with 60% solids was prepared using 70% by weight of the same fragrance oil and spray dried with inlet and outlet temperatures of 200°C and 100°C respectively, and otherwise using the same conditions as in Example 3. The efficiency of encapsulation was improved over that of Example 3 as indicated by surface oil value of 3.6% and total oil of 66.8%. The moisture content was 0.9%, which favored long range stability and good flow behavior.

The following examples 6 to 8 indicate the effects of increasing the solids level to 60%, with higher inlet and outlet temperatures and a pH of 5.3 to obtain crosslinking according to the invention.

### Example 6

An emulsion similar to that in Example 3 (60% oil loading but solids increased to 60% and the pH to 5.3), to which had been added 0.7% of the cross-linking agent sodium trimetaphosphate, was spray dried with inlet and outlet temperatures of 200°C and 100°C respectively, and otherwise treated by the same procedure as Example 3. It gave somewhat improved results as shown in Table 1.

### Example 7

An emulsion prepared by the procedure of Example 2 (60% oil loading, but with the solids increased to 60%, and after adjusting the pH of the water to 5.3 and adding 0.7% of sodium tri-meta phosphate to promote cross-linking of the matrix in situ during spray drying without raising the inlet and outlet temperatures (which were 190°C and 90°C) respectively. The procedure resulted in an encapsulation level comparable to that in Example 1, as indicated by a surface oil level of 1.0%, with a total oil level at 55.2% by weight. The results are summarized in the Table.

### Example 8

A 70% system of the same matrix material was obtained by adjusting the solids to 60% and the pH of the water to 5.3, and adding 0.7% of sodium tri-meta phosphate to promote cross-linking of the matrix in situ during spray drying. Good efficiency of encapsulation was indicated by further reduction in extractable oil to 4.2% in extended surface oil after 72 hrs. Comparable results were also obtained by repeating the example with the oil replaced, in turn, with mandarin limonene, fresh aldehydic citrus, and aldehydic lemon.

**Table 1**

| Conditions | | | | | | | Powder Specifications | | |
|---|---|---|---|---|---|---|---|---|---|
| Comp. Ex.# | % Oil Load | pH | Temps. | | TO⁽¹⁾ | SO⁽²⁾ | H₂O | FSO⁽³⁾ | SO⁽⁴⁾ |
| | | | In | Out | | | | | |
| | | | | | | | | | |

| 50% Solids Emulsion | | | | | | | | | |
|---|---|---|---|---|---|---|---|---|---|
| 1 | 50 | 3 | 180 | 80 | 47.0 | 0.1 | 1.6 | ∼2.0 | 0.5 |
| 2 | 60 | 3 | 180 | 80 | 57.6 | 0.1 | 1.1 | 6.9 | 1.2 |
| 3 | 70 | 3 | 180 | 80 | 64.7 | 5.2 | 1.3 | 18.6 | 15.7% |

| 60% Solids Emulsion | | | | | | | | | |
|---|---|---|---|---|---|---|---|---|---|
| Ex.# | % Oil Load | pH | Temps. | | | | | | |
| | | | In | Out | | | | | |
| 4 | 60 | 3 | 200 | 100 | 59.2 | 0.4 | 1.7 | 4.6 | 2.3 |
| 5 | 70 | 3 | 200 | 100 | 66.8 | 3.6 | 0.9 | 13.6 | 8.4 |

| 60% & Cross-linking | | | | | | | | | |
|---|---|---|---|---|---|---|---|---|---|
| 6 | 50 | 5.3 | 200 | 100 | 47.7 | 0.1 | 1.0 | 5.3 | 0.8 |
| 7 | 60 | 5.3 | 200 | 100 | 55.2 | 1.0 | 1.2 | 4.7 | 1.2 |
| 8 | 70 | 5.3 | 200 | 100 | 65.1 | 2.6 | 0.6 | 8.2 | 5.7 |

| 3% Silicone Plasticizer | | | | | | | | | |
|---|---|---|---|---|---|---|---|---|---|
| 9 | 50 | 3 | 190 | 90 | 49.3 | 0.6 | 1.2 | 2.0 | |
| 10 | 60 | 3 | 190 | 90 | 58.6 | 2.4 | 0.6 | 4.8 | |

| | | | | | | | | | |
|---|---|---|---|---|---|---|---|---|---|
| (1) Total Oil | | | | | | | | | |
| (2) Surface oil | | | | | | | | | |
| (3) Surface oil obtained after a friability test; an indication of the strength of the matrix. | | | | | | | | | |
| (4) Extended surface oil; results indicate strengthening of matrix upon in situ cross-linking during spray drying. | | | | | | | | | |

### Example 9

This sample was prepared following the procedure of Comparative Example 1, with the exception that the final product contained 3% Silicone-based emulsifier. This silicone-based emulsifier increased the pliability of the matrix.

### Example 10

This sample was prepared using the same procedure as Example 9. However, the percent of oil load was increased to 60% by weight from 50%.

**Table 2**

| Analysis Results | | | | | | | | | |
|---|---|---|---|---|---|---|---|---|---|
| Ex.# | % Oil Load | pH | Temps. | | TO⁽¹⁾ | SO⁽²⁾ | H₂O | FSO⁽³⁾ | SO₍₄₎ |
| | | | In | Out | | | | | |
| 9 | 50 | 3 | 190 | 90 | 49.3 | 0.6 | 1.2 | 2.0 | |
| 10 | 60 | 3 | 190 | 90 | 58.6 | 2.4 | 0.6 | 4.8 | |

## Claims

1. A method for encapsulating oils such as fragrance or flavoring oils in a water-sensitive cellular solid matrix, comprising drying an aqueous emulsion containing the oil to be encapsulated, a non-cross-linked lipophilically modified starch that undergoes cross-linking under the drying conditions, and a polyhydroxy compound that forms with the polysaccharide material a continuous aqueous phase in which oil is dispersible as a discontinuous phase.

2. A method for encapsulating oils as claimed in claim 1, in which the modified starch is an ungelatinized starch acid esters of a substituted dicarboxylic acid having the formula: 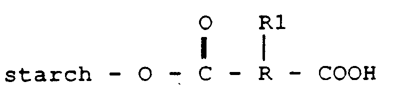 in which R is a radical selected from the class consisting of dimethylene and trimethylene and R1 is a hydrocarbon substituent selected from the class consisting of alkyl, alkenyl, aralkyl and groups, the ungelatinized starch-acid esters being the reaction product of an ungelatinized starch and, a substituted cyclic dicarboxylic acid anhydride having the formula: 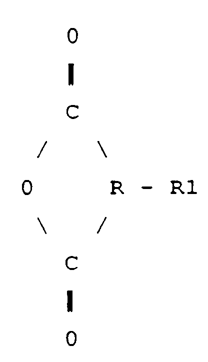 in which R and R1 represent the defined substituent groups in a water-sensitive cellular solid matrix, comprising drying an emulsion of the oil and a non-cross-linked lipophilically modified starch that undergoes cross-linking under the drying conditions, the amount of the oil encapsulated in the matrix being greater than 60% by weight of the oil and matrix combination.

3. A method for encapsulating oils as claimed in claim 1 in which the starch is selected from the group consisting of succinate starch, substituted succinate starch, linoleate starch, and substituted linoleate starch.

4. A method for encapsulating oils as claimed in claim 2 in which the starch is selected from the group consisting of succinate starch, substituted succinate starch, linoleate starch, and substituted linoleate starch.

5. A method for encapsulating oils as claimed in claim 1, in which the emulsion contains a cross-linking agent.

6. A method for encapsulating oils as claimed in claim 5, in which the cross-linking agent is selected from the group consisting of sodium trimetaphosphate, glutaraldehyde, acetaldehyde, and epichlorohydrin.

7. A method for encapsulating oils as claimed in claim 1, in which the emulsion also contains a silicone-based emulsifier that increases the pliability of the matrix.

8. A method for encapsulating oils as claimed in claim 1, in which from 10 % to 90% of the modified starch and from 90% to 10% of the polyhydroxy compound, based on the total solids weight, are dissolved with agitation at high speed in an amount of water such that the emulsion contains at least 40% solids.

9. A method for encapsulating oils as claimed in claim 9, in which the emulsion contains at least 60% solids and the inlet and outlet spray drying temperatures are200 and 100°C.

10. A method for encapsulating oils as claimed in claim 1, in which the pH of the emulsion is adjusted to 4.5 to 5.5 before the drying stage when crosslinking occurs.

11. A method for encapsulating oils as claimed in claim 1, in which more than 50% of the crosslinkable bonds of the polysaccharide are crosslinked.

12. A method for encapsulating oils as claimed in claim 1, in which the emulsion contains from 10 parts to 70 parts by weight of the oil.

13. A method for encapsulating oils as claimed in claim 1, in which the encapsulation matrix contains from about 5 to about 80% by volume of the oil.

14. A method for encapsulating oils as claimed in claim 1, in which the encapsulation matrix contains at least about 30% by volume of the matrix.

15. A method for encapsulating oils as claimed in claim 1, in which the encapsulation matrix contains more than 60% by weight of the oil.

## Patentansprüche

1. Verfahren zum Verkapseln von Ölen, wie Duft- oder Aromaölen, in eine wasserempfindliche, feste, zelluläre Matrix, umfassend das Trocknen einer wäßrigen Emulsion, die das zu verkapselnde Öl, eine unvernetzte, lipophil modifizierte Stärke, die beim Trocknen vernetzt und eine Polyhydroxyverbindung enthält, die zusammen mit dem Polysaccharidmaterial eine kontinuierliche wäßrige Phase bildet, in der Öl als diskontinuierliche Phase dispergierbar ist.

2. Verfahren zum Verkapseln von Ölen in einer wasserempfindlichen, festen, zellulären Matrix nach Anspruch 1, wobei die modifizierte Stärke ein nicht gelatinierter Stärke-Säureester einer substituierten Dicarbonsäure der Formel: 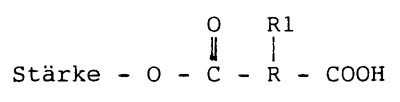 ist, in der R eine aus Dimethylen und Trimethylen ausgewählte Gruppe ist und R1 ein aus Alkyl-, Alkenyl-, Aralkylresten ausgewählter Kohlenwasserstoffsubstituent ist, wobei der nicht gelatinierte Stärke-Säureester das Reaktionsprodukt einer nicht gelatinierten Stärke und eines substituierten cyclischen Dicarbonsäureanhydrids der Formel: 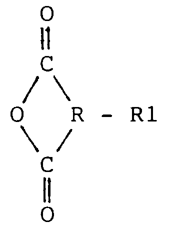 ist, in der R und R1 die definierten Substituentgruppen darstellen, umfassend das Trocknen einer Emulsion aus dem Öl und einer unvernetzten, lipophil modifizierten Stärke, die beim Trocknen vernetzt, wobei die Menge des in der Matrix verkapselten Öls mehr als 60 Gew.% der Kombination aus Öl und Matrix beträgt.

3. Verfahren zum Verkapseln von Ölen nach Anspruch 1, wobei die Stärke aus Bernsteinsäure-Stärkeestern, substituierten Bernsteinsäure-Stärkeestern, Linolsäure-Stärkeestern und substituierten Linolsäure-Stärkeestern ausgewählt ist.

4. Verfahren zum Verkapseln von Ölen nach Anspruch 2, wobei die Stärke aus Bernsteinsäure-Stärkeestern, substituierten Bernsteinsäure-Stärkeestern, Linolsäure-Stärkeestern und substituierten Linolsäure-Stärkeestern ausgewählt ist.

5. Verfahren zum Verkapseln von Ölen nach Anspruch 1, wobei die Emulsion ein Vernetzungsmittel enthält.

6. Verfahren zum Verkapseln von Ölen nach Anspruch 5, wobei das Vernetzungsmittel aus Natriumtrimetaphosphat, Glutaraldehyd, Acetaldehyd und Epichlorhydrin ausgewählt ist.

7. Verfahren zum Verkapseln von Ölen nach Anspruch 1, wobei die Emulsion weiterhin einen Emulgator auf Siliconbasis enthält, der die Geschmeidigkeit der Matrix erhöht.

8. Verfahren zum Verkapseln von Ölen nach Anspruch 1, wobei 10 bis 90% der modifizierten Stärke und 90 bis 10% der Polyhydroxyverbindung, bezogen auf das Gesamtgewicht der Feststoffe, unter Rühren mit hoher Geschwindigkeit in einer derartigen Menge Wasser gelöst werden, daß die Emulsion zumindest 40% Feststoffe enthält.

9. Verfahren zum Verkapseln von Ölen nach Anspruch 9, wobei die Emulsion zumindest 60% Feststoffe enthält und die Eingangs- und Ausgangstemperaturen der Sprühtrocknung bei 200 und 100°C liegen.

10. Verfahren zum Verkapseln von Ölen nach Anspruch 1, wobei der pH-Wert der Emulsion vor dem Trocknen unter Vernetzen auf 4,5 bis 5,5 eingestellt wird.

11. Verfahren zum Verkapseln von Ölen nach Anspruch 1, wobei mehr als 50% der vernetzbaren Bindungen des Polysaccharids vernetzt sind.

12. Verfahren zum Verkapseln von Ölen nach Anspruch 1, wobei die Emulsion 10 bis 70 Gewichtsteile des Öls enthält.

13. Verfahren zum Verkapseln von Ölen nach Anspruch 1, wobei die verkapselnde Matrix etwa 5 bis etwa 80 Volumenprozent des Öls enthält.

14. Verfahren zum Verkapseln von Ölen nach Anspruch 1, wobei die verkapselnde Matrix zumindest etwa 30 Volumenprozent der Matrix enthält.

15. Verfahren zum Verkapseln von Ölen nach Anspruch 1, wobei die verkapselnde Matrix mehr als 60 Gewichtsprozent des Öls enthält.

## Revendications

1. Procédé pour encapsuler des huiles, comme des parfums et des huiles ou essences aromatisantes dans une matrice solide cellulaire, sensible à l'eau, caractérisé en ce que l'on sèche une émulsion aqueuse contenant l'huile à encapsuler, un amidon modifié lipophiliquement et non réticulé qui subit une réticulation dans les conditions de séchage, et un composé polyhydroxylé qui forme avec la matière polysaccharidique une phase aqueuse continue, dans laquelle l'huile ou l'essence est dispersible sous forme d'une phase discontinue.

2. Procédé d'encapsulation d'huiles suivant la revendication 1, dans lequel l'amidon modifié est un ester acide d'amidon non gélatinisé d'un acide dicarboxylique substitué répondant à la formule : 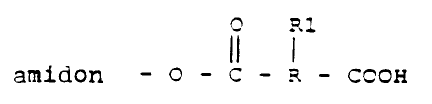 dans laquelle R représente un radical choisi dans la classe formée par le diméthylène et le triméthylène et R1 représente un substituant hydrocarboné choisi dans le groupe formé par les radicaux alkyle, alcényle, aralkyle, l'ester acide d'amidon non gélatinisé étant le produit de la réaction d'un amidon non gélatinisé et d'un anhydride d'acide dicarboxylique cyclique substitué répondant à la formule : 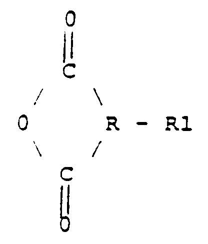 dans laquelle R et R1 représentent les radicaux substituants définis, dans une matrice solide cellulaire et sensible à l'eau, caractérisé en ce que l'on sèche une émulsion de l'huile et d'un amidon modifié lipophiliquement et non réticulé qui subit une réticulation dans les conditions du séchage, la quantité de l'huile encapsulée dans la matrice étant supérieure à 60% en poids de la combinaison huile et matrice.

3. Procédé d'encapsulation d'huiles suivant la revendication 1, caractérisé en ce que l'amidon est choisi dans le groupe formé par l'amidon succinate, l'amidon succinate substitué, l'amidon linoléate et l'amidon linoléate substitué.

4. Procédé d'encapsulation d'huiles suivant la revendication 2, caractérisé en ce que l'on choisit l'amidon dans le groupe formé par l'amidon succinate, l'amidon succinate substitué, l'amidon linoléate et l'amidon linoléate substitué.

5. Procédé d'encapsulation d'huiles suivant la revendication 1, caractérisé en ce que l'émulsion contient un agent de réticulation.

6. Procédé d'encapsulation d'huiles suivant la revendication 5, caractérisé en ce que l'on choisit l'agent de réticulation dans le groupe formé par le trimétaphosphate, le glutaraldéhyde, l'acétaldéhyde et l'épichlorhydrine.

7. Procédé d'encapsulation d'huiles suivant la revendication 1, caractérisé en ce que l'émulsion contient aussi un émulsif à base de silicone qui augmente la souplesse ou flexibilité de la matrice.

8. Procédé d'encapsulation d'huiles suivant la revendication 1, caractérisé en ce que de 10% à 90% de l'amidon modifié et de 90% à 10% du composé polyhydroxylé, sur base du poids des solides totaux, sont dissous sous agitation à grande vitesse dans une quantité d'eau telle qu'une émulsion contienne au moins 40% de solides.

9. Procédé d'encapsulation d'huiles suivant la revendication 8, caractérisé en ce que l'émulsion contient au moins 60% de solides et les températures de séchage par pulvérisation d'entrée et de sortie sont de 200 et de 100°C.

10. Procédé d'encapsulation d'huiles suivant la revendication 1, caractérisé en ce que le pH de l'émulsion est ajusté à 4,5 à 5,5 avant l'étape de séchage lorsque la réticulation s'opère.

11. Procédé d'encapsulation d'huiles suivant la revendication 1, caractérisé en ce que plus de 50% des liaisons réticulables du polysaccharides sont réticulés.

12. Procédé d'encapsulation d'huiles suivant la revendication 1, caractérisé en ce que l'émulsion contient de 10 parties à 70 parties en poids de l'huile.

13. Procédé d'encapsulation d'huiles suivant la revendication 1, caractérisé en ce que la matrice d'encapsulation contient d'environ 5 à environ 80% en volume de l'huile.

14. Procédé d'encapsulation d'huiles suivant la revendication 1, caractérisé en ce que la matrice d'encapsulation contient au moins environ 30% en volume de la matrice.

15. Procédé d'encapsulation d'huiles suivant la revendication 1, caractérisé en ce que la matrice d'encapsulation contient plus de 60% en poids de l'huile.
